(19) **Europäisches Patentamt**

**European Patent Office**

**Office européen des brevets**

(11) **EP 0 941 985 B1**

(12) **EUROPÄISCHE PATENTSCHRIFT**

(45) Veröffentlichungstag und Bekanntmachung des Hinweises auf die Patenterteilung:
**06.03.2002 Patentblatt 2002/10**

(51) Int Cl.⁷: **C07C 209/36**, C07C 209/38, C07C 211/55

(21) Anmeldenummer: **99104230.0**

(22) Anmeldetag: **03.03.1999**

(54) **Verfahren zur Herstellung von 4-Aminodiphenylamin**

Process for the preparation of 4-aminodiphenylamine

Procédé pour la préparation de la 4-amino-diphénylamine

(84) Benannte Vertragsstaaten:
**BE DE FR GB IT**

(30) Priorität: **13.03.1998 DE 19810929**

(43) Veröffentlichungstag der Anmeldung:
**15.09.1999 Patentblatt 1999/37**

(73) Patentinhaber: **BAYER AG**
**51368 Leverkusen (DE)**

(72) Erfinder:
• **Ooms, Pieter, Dr.**
**47800 Krefeld (DE)**
• **Giera, Henry, Dr.**
**51429 Bergisch Gladbach (DE)**

(56) Entgegenhaltungen:
**EP-A- 0 784 049       EP-A- 0 895 983**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die Erfindung betrifft ein Verfahren zur Herstellung von 4-Aminodiphenylamin (4-ADPA) durch Hydrierung von Nitrosobenzol mit Wasserstoff in Gegenwart von Hydrierkatalysatoren und Fluoriden.

4-ADPA ist ein wichtiges Zwischenprodukt für Alterungsschutzmittel und Stabilisatoren in der Gummi- und Polymerindustrie (Kirk-Othmer, Encyclopedia of Chemical Technology, 4th Edition, 1992, Vol. 3, S. 424-447 und S 448-456; Ullmann's Encyclopedia of Industrial Chemistry, 5th Edition, Vol A3, 1985, S 91-111).

4-Aminodiphenylamin kann nach verschiedenen Methoden hergestellt werden. Eine Möglichkeit 4-ADPA herzustellen, ist die zweistufige (Zwischenprodukt 4-Nitrodiphenylamin) Umsetzung von Anilin bzw. Anilinderivaten mit p-Nitrochlorbenzol in Gegenwart eines Säureakzeptors oder eines Neutralisierungsmittels und gegebenenfalls in Gegenwart eines Katalysators. Die Herstellung nach dieser Methode ist beispielsweise beschrieben in DE-A 35 01 698, DE-A 18 56 63, US 4 670 595, US 4 187 249, US 468 3332 und US 4 187 248. Ein Nachteil eines solchen Verfahrens ist, daß die dabei entstehenden Salze mit beträchtlichen Kosten entsorgt werden müssen. Daher hat man Anilin bzw. entsprechende Anilinderivate mit Nitrobenzol in Gegenwart von Tetraalkylammoniumhydroxiden und in Gegenwart kontrollierter Mengen an protischen Materialien umgesetzt. 4-ADPA wurde dabei in einer befriedigenden Menge erhalten (siehe WO 93/00 324 und WO 93 24 450). Nach US 5 420 354 kann man 4-ADPA durch Umsetzung von Anilin, Nitrobenzol und Wasserstoff in Gegenwart eines Hydrierkatalysators, Hydrierinhibitors und sauren Co-Katalysators allerdings in wenig befriedigenden Ausbeuten erhalten. In US 5 574 187 wird beschrieben, daß man durch Umsetzung von Anilin mit Nitrosobenzol oder Phenylhydroxylamin in Gegenwart von Säuren 4-ADPA erhalten kann.

Diese Verfahren haben jedoch den Nachteil, daß zwei verschiedene Edukte, die in vorgelagerten separaten Verfahrensschritten hergestellt werden müssen, eingesetzt werden, was weniger wirtschaftlich ist.

Es ist auch bekannt, daß die Hydrierung von Nitrosobenzol über heterogene Katalysatoren hauptsächlich Anilin und Hydrazobenzol liefert. 4-ADPA wird als Produkt nicht erwähnt (Chem. Ind. 1994, Catalysis of Organic Reactions. S. 137-149).

Überraschenderweise wurde nun gefunden, daß man durch Hydrierung von Nitrosobenzol in Gegenwart von Fluoriden und heterogenen Katalysatoren 4-ADPA in technisch brauchbaren Ausbeuten erhalten kann.

Gegenstand der vorliegenden Erfindung ist daher ein Verfahren zur Herstellung von 4-Aminodiphenylamin, das dadurch gekennzeichnet ist, daß man Nitrosobenzol oder Gemische aus Nitrosobenzol und Nitrobenzol mit Wasserstoff in Gegenwart von Fluoriden und heterogenen Katalysatoren sowie in Gegenwart von inerten aprotischen Lösungsmitteln bei, Temperaturen von 0 bis 200°C und Drücken von 0,1 bis 150 bar hydriert.

Als für das erfindungsgemäße Verfahren geeignete Fluoride kommen in Frage anorganische Fluoride, wie Alkalimetallfluoride, Erdalkalimetallfluoride, sowie die entsprechenden Fluoride der Elemente 58 bis 71 des Periodensystems der Elemente (nach IUPAC, neu). Beispielsweise werden genannt: Die Fluoride von Natrium, Kalium, Lithium, Caesium, Rubidium, Magnesium, Calcium, Barium, Lanthan und/oder Cer, insbesondere die Fluoride von Lithium, Natrium, Kalium, Caesium, ganz besonders bevorzugt Kaliumfluorid, Caesiumfluorid, Natriumfluorid.

Weiterhin kommen organische Basen in Frage, wie beispielsweise quaternäre Alkylammoniumfluoride ($NR_4^+F^-$ mit R unabhängig voneinander für Alkyl, Aryl oder Aralkyl mit 1 bis 8 Kohlenstoffatomen). Als Beispiele seien genannt: Tetramethylammoniumfluorid, Tetraethylammoniumfluorid, Tetrapropylammoniumfluorid, Tetrabutylammoniumfluorid, Tetrapentylammoniumfluorid, Tetrahexylammoniumfluorid, Tetraheptylammoniumfluorid, Tetraoctylammoniumfluorid, Methyltributylammoniumfluorid, Methyltripropylammoniumfluorid, Methyltriethylammoniumfluorid, Trimethylbenzylammoniumfluorid. Besonders bevorzugt sind Tetramethylammoniumfluorid, Tetraethylammoniumfluorid, Tetrapropylammoniumfluorid und Tetrabutylammoniumfluorid. Ganz besonders bevorzugt wird Tetramethylammoniumfluorid, Tetraethylammoniumfluorid und Tetrabutylammoniumfluorid eingesetzt.

Es können auch beliebige Mischungen der vorgenannten Fluoride eingesetzt werden.

Solche Fluoride und Herstellungsverfahren für solche Verbindungen aus Vorprodukten wie beispielsweise Oxiden, Hydroxiden, Bromiden oder Chloriden sind in Kirk-Othmer, Encyclopedia of Chemical Technology, Auflage, Bd. 9, S. 527 ff., New York 1966, Houben-Weyl, Methoden der organischen Chemie, Bd. E. 16a, Tl 2, S. 1015 ff, Stuttgart 1990 und Chemical Reviews 80, (1980), S. 429 ff., beschrieben.

Weiterhin ist es möglich, die anorganischen Fluoride in Verbindung mit Phasentransferkatalysatoren einzusetzen. Geeignete Phasentransferkatalysatoren sind beispielsweise beschrieben in W.E. Keller, Fluka-Kompendium, Bd. 1, 2, 3, Georg Thieme Verlag, Stuttgart 1986, 1987, 1992. Beispielsweise können die zuvor erwähnten Fluoride mit Kronenether, wie 18-Krone-6 oder quartären Ammoniumverbindungen, zusammen eingesetzt werden.

Die erfindungsgemäß einzusetzenden Fluoride können einen Wassergehalt von bis zu 6 mol Wasser, bevorzugt bis zu 5 mol Wasser, besonders bevorzugt bis zu 4 mol Wasser, bezogen auf ein Mol Fluorid, besitzen.

Die erfindungsgemäßen Fluoride können dem Reaktionsgemisch in fester Form, als Schmelze oder als Lösung in einem aprotischen Lösungsmittel oder in einer Mischung aus Nitrosobenzol und einem oder mehreren aprotischen Lösungsmitteln zugesetzt werden.

Die Fluoride werden dabei in einer Menge von 0,01 bis 3, bevorzugt 0,1 bis 2, insbesondere 0,3 bis 1,5 Äquivalenten pro Mol Nitrosobenzol eingesetzt.

Erfindungsgemäß können die obengenannte Fluoride in reiner Form oder aufgebracht auf einem Träger wie beispielsweise $Al_2O_3$, $SiO_2$ oder Polymerharz eingesetzt werden.

Als inerte aprotische Lösungsmittel kommen aromatische Kohlenwasserstoffe mit 6 bis 20 Kohlenstoffatomen, lineare oder cyclische Ether mit bis zu 5 Sauerstoffatomen und 2 bis 16 Kohlenstoffatomen, aromatische halogenierte Kohlenwasserstoffe mit 6 bis 20 Kohlenstoffatomen sowie Amide mit 1 bis 10 Kohlenstoffatomen in Frage. Selbstverständlich können die erwähnten Lösungsmittel im Gemisch untereinander eingesetzt werden. Als geeignete Lösungsmittel werden insbesondere genannt: Benzol, Toluol, Xylol, tert.-Butylmethylether, tert.-Amylmethylether, Diisopropylether, Diethylenglykoldimethylether, Glykoldimethylether, Dioxan, Tetrahydrofuran, Diamylether, Chlorbenzol, Dichlorbenzol, Dimethylformamid, Dimethylacetamid und N-Methylpyrolidinon. Bevorzugt werden eingesetzt, Toluol, Xylol, Glykoldimethylether, Dioxan, Tetrahydrofuran, tert.-Butylmethylether, Diisopropylether, Diethylenglykoldimethylether, insbesondere tert.-Butylmethylether, Diethylenglykoldimethylether. Glykoldimethylether, Dioxan, Tetrahydrofuran und Toluol. Die Menge an Lösungsmittel ist für das erfindungsgemäße Verfahren nicht kritisch und hängt insbesondere ab von der Reaktionstemperatur und von der Art und Menge der eingesetzten Fluoride und Katalysatoren. Üblicherweise werden die Lösungsmittel in Mengen von 1 bis 99 Gew.-%. bevorzugt 5 bis 95 Gew.-%, besonders bevorzugt 15 bis 90 Gew.-%, bezogen auf die Gesamtmenge der Reaktionsmischung, eingesetzt

Als heterogene Katalysatoren eignen sich für das erfindungsgemäße Verfahren praktisch alle heterogenen Katalysatoren, die für Hydrierungsreaktionen bekannt sind. Die erfindungsgemäßen Katalysatoren umfassen Metalle der 8-10 Gruppe des Periodensystems (nach IUPAC, neu) oder Kupfer und/oder Chrom auf geeignetem Träger mit einem Metallgehalt von 0,01 bis 50 Gew.-%, bevorzugt 0,1 bis 10 Gew.-%, bezogen auf das Gesamtgewicht des Katalysators. Erfindungsgemäß können Katalysatoren eingesetzt werden, die eines oder mehrere der obengenannten Metalle enthalten. Die angegebenen Gewichtsanteile gelten bei Anwesenheit mehrerer Elemente für die Summe der Einzelanteile. Bevorzugte Metalle sind insbesondere Platin, Palladium, Rhodium und Ruthenium, besonders bevorzugt sind Platin. Palladium und Rhodium. Weitere bevorzugte Katalysatoren sind Raney-Nickel und geträgerte Nikkelkatalysatoren.

Erfindungsgemäß können auch die obengenannten Metalle oder ihre Verbindungen in reiner Form als Feststoff eingesetzt werden. Als Beispiele für ein Metall in reiner Form seien Palladium- und Platinschwarz genannt.

Die Herstellung der erfindungsgemäßen Katalysatoren kann nach den verschiedensten Methoden erfolgen, die dem Fachmann bekannt sind. So können Lösungen einer oder mehrerer der genannten Metallverbindungen, beispielsweise durch Tränken, Adsorption, Tauchen, Sprühen, Imprägnieren und Ionenaustausch auf den erfindungsgemäß einzusetzenden Katalysatorträger gebracht werden. Dem Katalysator können in bekannter Art und Weise weitere Elemente zugefügt werden. Es ist weiterhin möglich, ein oder mehrere der genannten Metalle durch Fällung mit einer Base auf dem Träger zu fixieren. Als Base kommen z.B. (Erd-)Alkalimetallhydroxide in Frage. Ein oder mehrere Metalle können sowohl in beliebiger Reihenfolge nacheinander als auch gleichzeitig auf den Träger gebracht werden. Eine spezielle Ausführungsform der Erfindung beinhaltet das Aufbringen des Metalls durch Fällung eines Metallhalogenids oder einer Metallhalogenid-Komplexverbindung mit einer geeigneten Base und Reduktion der Metallverbindung zum Metall. Bei der Herstellung der Träger mittels eines Sol-Gel-Verfahrens können in einer Ausführungsform Lösungen einer oder mehrerer der genannten Metallverbindungen in dem Fachmann bekannter Weise bereits dem Sol zugesetzt werden.

Geeignete Materialien für die erfindungsgemäße Verwendung als Katalysatorträger sind alle technisch üblichen Katalysatorträger auf der Basis Kohlenstoff, Elementoxiden. Elementcarbiden oder Elementsalzen in verschiedenen Anwendungsformen. Beispiele für kohlenstoffhaltige Träger sind Koks, Graphit, Ruß oder Aktivkohlen. Beispiele für Elementoxid-Katalysatorträger sind $SiO_2$ (natürliche oder synthetische Kieselsäure, Quarz), $Al_2O_3$ ($\alpha$-, $\gamma$-$Al_2O_3$), Tonerden, natürliche und synthetische Alumosilicate (Zeolithe), Schichtsilikate, wie Bentonit und Montmorillonit, $TiO_2$ (Rutil, Anatas), $ZrO_2$, $MgO$ oder $ZnO$. Beispiele für Elementcarbide und -salze sind $SiC$, $AlPO_4$, $BaSO_4$, $CaCO_3$. Grundsätzlich können sowohl synthetische Materialien als auch Träger aus natürlichen Quellen, wie z.B. Bimsstein, Kaolin, Bleicherden. Bauxite, Bentonite, Kieselgur, Asbest oder Zeolithe, verwendet werden.

Weitere brauchbare Träger für die erfindungsgemäß einsetzbaren Katalysatoren sind Elementmischoxide und Oxidhydrate von Elementen der Gruppen 2 bis 16 des Periodensystems sowie der Seltenerdmetalle (Atomnummern 58 bis 71), bevorzugt aus den Elementen Al, Si, Ti, Zr, Zn, Mg, Ca, Zn, Nb und Ce, die u. a. auf dem Weg über mechanische Vermischungen, gemeinsame Fällungen von Salzen oder über Cogele aus Salzen und/oder Alkoxiden hergestellt werden können, wie dies dem Fachmann bekannt ist.

Beispiele für Mischoxide sind Magnesiumaluminiumoxide (Hydrotalcite).

Die Träger können sowohl im Sinne chemisch einheitlicher Reinsubstanzen als auch im Gemisch eingesetzt werden. Für die erfindungsgemäße Verwendung als Katalysatorträger eignen sich sowohl stückige als auch pulverförmige Materialien. Für den Fall der Anordnung des Träger-Katalysators als Festbett wird der Träger vorzugsweise als Formkörper, z.B. als Kugeln, Zylinder, Stäbchen, Hohlzylinder. Ringe usw. eingesetzt. Wahlweise können Katalysatorträger weiter durch Extrudieren, Tablettieren, gegebenenfalls unter Zumischen weiterer Katalysatorträger oder Bindemittel, wie $SiO_2$ oder $Al_2O_3$, und Kalzinieren modifiziert werden. Die innere Oberfläche der Träger (BET-Oberfläche) liegt bei 1 bis 2 000 m$^2$/g, bevorzugt bei 10 bis 1 600 m$^2$/g, ganz besonders bevorzugt bei 20 bis 1 500 m$^2$/g. Darstellung und Weiterverarbeitung der erfindungsgemäß verwendeten Katalysatorträger sind dem Fachmann wohl bekannt und Stand der Technik.

Bevorzugt werden Aktivkohlen und Si-, Al-, Mg-, Zr- und Ti-haltige Materialien als Trägermaterialien eingesetzt, besonders bevorzugt sind Aktivkohle sowie silizium-, magnesium- und aluminiumhaltige Träger.

Die erfindungsgemäßen Katalysatoren können in diskontinuierlichen Verfahrensvarianten in Mengen von 0,01 bis 20 Gew.-% bezogen auf eingesetztes Nitrosobenzol verwendet werden, bevorzugt in Mengen von 0,01 bis 10 Gew.-%. Bei kontinuierlicher Durchführung der Reaktion, beispielsweise in einem Rührkessel mit einem pulverförmigen Katalysator oder in der Rieselphase am Festbettkatalysator, können Belastungen von 0,01 bis 500 g Nitrosobenzol pro g Katalysator und Stunde verwendet werden. Bevorzugt sind Belastungen von 0,02 bis 300 g Nitrosobenzol pro g Katalysator und Stunde.

In das erfindungsgemäße Verfahren können Nitrosobenzol oder Nitrosobenzol/ Nitrobenzolgemische, wie sie beispielsweise bei der Herstellung von Nitrosobenzol aus Nitrobenzol anfallen, eingesetzt werden. Der Gehalt an Nitrosobenzol kann beispielsweise 0,5 bis 99 %. bevorzugt 0,5 bis 98 %, besonders bevorzugt 1 bis 97 % sein.

Die Reaktionstemperaturen bei dem erfindungsgemäßen Verfahren betragen 0 bis 200°C, insbesondere 25 bis 150°C; die Drücke (Wasserstoffdruck) liegen bei 0.1 bis 150 bar, insbesondere 0.5 bis 70 bar, ganz besonders bevorzugt 1 bis 50 bar.

Es ist möglich, die Reaktion bei einer konstanten Temperatur und bei konstantem Wasserstoffdruck durchzuführen; Wasserstoffdruck und Temperatur können aber auch im Verlauf der Reaktion geändert werden bzw. in verschiedenen Reaktoren verschieden sein. Bei der diskontinuierlichen Durchführung können Nitrosobenzol, Katalysator, Lösungsmittel und Fluorid in beliebiger Reihenfolge in den Reaktor eingespeist werden. Die Wasserstoffzufuhr kann nach einer bestimmten zugeführten Menge abgebrochen und gegebenenfalls später wieder fortgesetzt werden.

Kontinuierliche Verfahrensvarianten sind beispielsweise die Hydrierung in der Sumpfphase mit einem pulverförmigen suspendierten Katalysator (Slurry), die Hydrierung in der Rieselphase am Festbett-Katalysator oder die Hydrierung mit einem suspendierten Katalysator in einer Blasensäule. Die Reaktion kann in den dem Fachmann bekannten Apparaten zur Kontaktierung von Fest-, Flüssig- und Gasphasen durchgeführt werden. Insbesondere kommen hier Rührkessel. Umpumpreaktoren, Busreaktoren, im Gleich- oder Gegenstrom betriebene Blasensäulen oder Rieselphasenreaktoren oder Kaskaden dieser Reaktoren, in Frage, wobei die verschiedenen Reaktortypen auch gleichzeitig in einer Kaskade vorkommen können.

Wird der Katalysator als Pulver in der Sumpfphase eingesetzt, sind zur Vermischung der Reaktionskomponenten, die zu verwendenden Rührbehälter mit dafür brauchbaren Rührern ausgestattet. Möglich sind der Einsatz von Flügel-, MIG-, Propeller-, Anker- oder Begasungsrührer.

Die darüber hinaus bei dem erfindungsgemäßen Verfahren anfallenden Stoffe sind Zwischenprodukte der Hydrierung von Nitrosobenzol zu Anilin und lassen sich restlos in Anilin überführen, welches ebenfalls ein wertvolles Ausgangsprodukt für die Synthese vieler industrieller Endprodukte ist.

## Beispiele

Folgende Beispiele demonstrieren die Durchführbarkeit der Reaktion bei verschiedenen Temperaturen und unter Verwendung verschiedener Katalysatoren, Fluoride und Lösungsmittel. Die Reaktionsprodukte wurden gaschromatografisch (Durabond DB-5-MS; 30 m x 0,25 mm ID) mit dem internen Standard n-Tridekan bzw. mittels quantitativer HPLC analysiert. Der Umsatz an Nitrosobenzol war in allen beschriebenen Versuchen vollständig. Aufarbeitung und Probenpräparation wurde unter Stickstoff durchgeführt.

Die eingesetzten Fluoride und Katalysatoren sind käufliche Produkte oder wurden wie im folgenden beschrieben, hergestellt.

## Herstellung des Katalysators Pt/Aktivkohle

475 g Aktivkohle (Norit-B-Supra, Fa. Norit) wurden in 2 600 ml entionisiertem Wasser aufgeschlämmt, die Mischung auf 50°C erwärmt und mit einer Lösung von 87,5 g Natriumformiat in 400 ml entionisiertem Wasser versetzt. In 30 Minuten wurde eine Mischung aus 100 g einer $H_2PtCl_4$-Lösung (25 Gew.-% Pt) und 400 ml entionisiertem Wasser zugetropft und eine Stunde bei 50°C nachgerührt. Anschließend wurde der Katalysator abgesaugt, gewaschen und im Vakuum bei 60°C getrocknet.

## Beispiele 1 bis 12 (Hydrierungen unter Normaldruck)

## Beispiel 1

In einem mit Stickstoff gespülten 250 ml Planschlifftopf mit Begasungsrührer wurden 65 ml Diethylenglykoldimethylether, 17,4 g (0,055 mol) $(H_9C_4)_4NF.3H_2O$ der Firma Fluka und 0.5 g pulverförmigen 5 % Pd/C-Katalysator 3230 der Firma Engelhard vorgelegt und auf 80°C erwärmt. Nach Erreichen dieser Temperatur wurde der Stickstoff bei Normaldruck durch einen Wasserstoffstrom von 25 l/h ersetzt und gleichzeitig 5,89 g (0,055 mol) Nitrosobenzol in 10 ml Diethylenglykoldimethylether zugegeben. Nach 1 h wurde eine Probe entnommen, filtriert, mit Essigsäure neutralisiert und mittels quantitativer Gaschromatografie analysiert. Der Umsatz an Nitrosobenzol war vollständig. Die Ausbeute an 4-ADPA betrug 11.7 % (die %-Angaben sind mol-% bezogen auf Nitrosobenzol).

### Beispiel 2

Das Beispiel 1 wurde mit 0,5 g eines pulverförmigen 5 % Pt/C-Katalysators in 75 ml Monoglym als Lösungsmittel bei 40°C wiederholt. Nach 18 h Reaktionszeit betrug die Ausbeute an 4-ADPA 15,1 %.

### Beispiel 3

Das Beispiel 1 wurde mit demselben Katalysator und $(H_9C_4)_4NF$ in 75 ml Tetrahydrofuran der Firma Aldrich bei 40°C wiederholt. Nach 18 h Reaktionszeit betrug die Ausbeute an 4-ADPA 15,6 %.

### Beispiel 4

Das Beispiel 1 wurde mit demselben Katalysator in 75 ml Toluol als Lösungsmittel bei 80°C wiederholt. Nach 1 h Reaktionszeit betrug die Ausbeute an 4-ADPA 13,1 %.

### Beispiel 5

Das Beispiel 1 wurde mit demselben Katalysator bei 40°C wiederholt. Nach 90 min. Reaktionszeit betrug die Ausbeute an 4-ADPA 12.1 %.

### Beispiel 6

Das Beispiel 1 wurde mit 8,0 g 40 % $KF/Al_2O_3$ der Firma Aldrich bei 80°C wiederholt. Nach 2 h Reaktionszeit betrug die Ausbeute an 4-ADPA 1,7 %.

### Beispiel 7

Das Beispiel 1 wurde mit 8,4 g (0,055 mol) CsF der Firma Aldrich bei 80°C wiederholt. Nach 14 h Reaktionszeit betrug die Ausbeute an 4-ADPA 1 %.

### Beispiel 8

Das Beispiel 1 wurde mit 9,1 g (0,055 mol) $(H_3C)_4NF.4H_2O$ der Firma Fluka bei 80°C wiederholt.

Nach 30 min Reaktionszeit betrug die Ausbeute an 4-ADPA 1 %.

### Beispiel 9

Das Beispiel 1 wurde mit 10.2 g (0,055 mol) $(H_5C_2)_4NF.2H_2O$ der Firma Fluka bei 80°C wiederholt. Nach 2 h Reaktionszeit betrug die Ausbeute an 4-ADPA 9.3 %.

### Beispiel 10

Das Beispiel 1 wurde mit 10,3 g (0,055 mol) $H_5C_6CH_2(H_3C)_3NF.H_2O$ der Firma Fluka bei 80°C wiederholt. Nach 6 h Reaktionszeit betrug die Ausbeute an 4-ADPA 4,6 %.

### Beispiel 11

Das Beispiel 1 wurde mit 7 g Amberlyst 26 (F-Form), einem Fluorid auf Polymerträger der Firma Aldrich, bei 80°C wiederholt. Nach 90 min Reaktionszeit betrug die Ausbeute an 4-ADPA 1,4 %.

### Beispiel 12

Das Beispiel 1 wurde mit einer Mischung aus 2,36 g (0,022 mol) Nitrosobenzol und 2,71 g (0,022 mol) Nitrobenzol bei 80°C wiederholt. Nach 30 min Reaktionszeit betrug die Ausbeute an 4-ADPA 4,7 %.

### Patentansprüche

1. Verfahren zur Herstellung von 4-Aminodiphenylamin, **dadurch gekennzeichnet, daß** man Nitrosobenzol oder Gemische aus Nitrosobenzol und Nitrobenzol mit Wasserstoff in Gegenwart von Fluoriden und heterogenen Katalysatoren sowie in Gegenwart von inerten aprotischen Lösungsmitteln bei Temperaturen von 0 bis 200°C und Drücken von 0,1 bis 150 bar hydriert.

2. Verfahren nach Anspruch 1, **dadurch gekennzeichnet, daß** man bei Temperaturen von 25 bis 150°C und Drücken von 0.5 bis 70 bar hydriert.

3. Verfahren nach Anspruch 1, **dadurch gekennzeichnet, daß** man als Fluoride Alkalimetallfluoride, Erdalkalimetallfluoride, sowie die entsprechenden Fluoride der Elemente 58 bis 71 des Periodensystems der Elemente (nach IUPAC, neu) sowie quaternäre Alkylammoniumfluoride, gegebenenfalls aufgebracht auf einem Träger, einsetzt.

4. Verfahren nach Anspruch 1, **dadurch gekennzeichnet, daß** man die Fluoride in Mengen von 0,01 bis 3 Äquivalente pro Mol Nitrosobenzol einsetzt.

**5.** Verfahren nach Anspruch 1, **dadurch gekennzeichnet, daß** man als aprotische Lösungsmittel aromatische Kohlenwasserstoffe mit 6 bis 20 Kohlenstoffatomen, lineare oder cyclische Ether mit bis zu 5 Sauerstoffatomen und 2 bis 16 Kohlenstoffatomen, aromatische halogenierte Kohlenwasserstoffe mit 6 bis 20 Kohlenstoffatome sowie Amide mit 1 bis 10 Kohlenstoffatomen einsetzt.

**6.** Verfahren nach Anspruch 1, **dadurch gekennzeichnet, daß** man die inerten aprotischen Lösungsmittel in Mengen von 1 bis 99 Gew.-%. bezogen auf die Gesamtmenge der Reaktionsmischung, einsetzt.

**7.** Verfahren nach Anspruch 1, **dadurch gekennzeichnet, daß** man als heterogene Katalysatoren Metallc der 8. bis 10. Gruppe des Periodensystems (nach IUPAC, neu) oder Kupfer und/oder Chrom, gegebenenfalls aufgebracht auf einen Katalysatorträger, einsetzt.

**8.** Verfahren nach Anspruch 1, **dadurch gekennzeichnet, daß** man den Katalysator in diskontinuierlicher Fahrweise in Mengen von 0.01 bis 20 Gew.-%, bezogen auf eingesetztes Nitrosobenzol verwendet. oder in kontinuierlicher Fahrweise Belastungen von 0,01 bis 500 g Nitrosobenzol pro g Katalysator und Stunde einsetzt.

## Claims

**1.** A process for the preparation of 4-aminodiphenylamine, **characterised in that** nitrosobenzene or mixtures of nitrosobenzene and nitrobenzene are hydrogenated with hydrogen in the presence of fluorides and heterogeneous catalysts and in the presence of inert aprotic solvents at temperatures from 0 to 200°C and pressures from 0.1 to 150 bar.

**2.** A process according to claim 1, **characterised in that** hydrogenation is carried out at temperatures from 25 to 150°C and pressures from 0.5 to 70 bar.

**3.** A process according to claim 1, **characterised in that** the fluorides used are alkali metal fluorides, alkaline earth metal fluorides and the corresponding fluorides of the elements 58 to 71 of the periodic system of elements (according to IUPAC, new), and quaternary alkylammonium fluorides, optionally deposited on a support.

**4.** A process according to claim 1, **characterised in that** the fluorides are used in quantities from 0.01 to 3 equivalents per mole of nitrosobenzene.

**5.** A process according to claim 1, **characterised in that** the aprotic solvents used are aromatic hydrocarbons having 6 to 20 carbon atoms, linear or cyclic ethers having up to 5 oxygen atoms and 2 to 16 carbon atoms, aromatic halogenated hydrocarbons with 6 to 20 carbon atoms and amides having 1 to 10 carbon atoms.

**6.** A process according to claim 1, **characterised in that** the inert aprotic solvents are used in quantities from 1 to 99 wt.%, based on the total quantity of reaction mixture.

**7.** A process according to claim 1, **characterised in that** the heterogeneous catalysts used are metals of the 8th to 10th group of the periodic system (according to IUPAC, new) or copper and/or chromium, optionally deposited on a catalyst support.

**8.** A process according to claim 1, **characterised in that** the catalyst is used in a batchwise operation in quantities from 0.01 to 20 wt.%, based on nitrosobenzene used or, in a continuous operation, loadings from 0.01 to 500 g of nitrosobenzene per g of catalyst and per hour are used.

## Revendications

**1.** Procédé de préparation de 4-aminodiphénylamine, **caractérisé en ce que** l'on hydrogène du nitrosobenzène ou des mélanges de nitrosobenzène et de nitrobenzène avec de l'hydrogène en présence de fluorures et de catalyseurs hétérogènes, et en présence de solvants aprotiques inertes, à des températures de 0 à 200°C et sous des pressions de 0,1 à 150 bars.

**2.** Procédé selon la revendication 1, **caractérisé en ce que** l'on effectue l'hydrogénation à des températures de 25 à 150°C et sous des pressions de 0,5 à 70 bars.

**3.** Procédé selon la revendication 1, **caractérisé en ce que** l'on utilise comme fluorures des fluorures de métaux alcalins, des fluorures de métaux alcalino-terreux, et les fluorures correspondants des éléments 58 à 71 du système périodique des éléments (nouveau, selon l'UICPA) et des fluorures d'alkylammonium quaternaire, éventuellement appliqués sur un support.

**4.** Procédé selon la revendication 1, **caractérisé en ce que** l'on utilise les fluorures en des quantités de 0,01 à 3 équivalents par mol de nitrosobenzène.

**5.** Procédé selon la revendication 1, **caractérisé en ce que** l'on utilise comme solvants aprotiques des hydrocarbures aromatiques de 6 à 20 atomes de carbone, des éthers linéaires ou cycliques ayant jusqu'à 5 atomes d'oxygène et de 2 à 16 atomes de carbone, des hydrocarbures halogénés aromatiques de 6 à 20 atomes de carbone et des amides de 1 à 10 atomes de carbone.

6. Procédé selon la revendication 1, **caractérisé en ce que** l'on utilise les solvants aprotiques inertes en des quantités de 1 à 99 % en masse par rapport à la quantité totale du mélange réactionnel.

7. Procédé selon la revendication 1, **caractérisé en ce que** l'on utilise comme catalyseurs hétérogènes des métaux des groupes 8 à 10 du système périodique (nouveau, selon l'UICPA) ou du cuivre et/ou du chrome, éventuellement appliqués sur un support de catalyseur.

8. Procédé selon la revendication 1, **caractérisé en ce que** l'on utilise le catalyseur dans un mode opératoire discontinu en des quantités de 0,01 à 20 % en masse par rapport au nitrosobenzène utilisé, ou **en ce que**, dans un mode opératoire continu, on utilise des charges de 0,01 à 500 g de nitrosobenzène par g de catalyseur et par heure.